# EUROPEAN PATENT APPLICATION

(11) **EP 1 779 885 A1**
(43) Date of publication of application: **02.05.2007**
(21) Application number: 05763023.8
(22) Date of filing: 24.06.2005
(51) Int. Cl.: A61M 15/00

(54) **ODOUR-SAMPLING DEVICE**

(30) Priority: 25.06.2004 ES 200401558
(71) Applicant: SIREROL CASABO, Dario, 08005 Barcelona (ES); ROVIRA MONTESINOS, Enric, 08296 CASTELLBELL I EL VILAR (ES)
(72) Inventor: SIREROL CASABO, Dario, E-08005 BARCELONA (ES)
(74) Representative: Carpintero Lopez, Francisco
(86) International application number: PCT/ES2005/000361
(87) International publication number: WO 2006/003219

(57) **Abstract**

The present invention relates to a device for testing odors, comprising a body (1) internally incorporating an emanating material (2) emanating a characteristic odor and is formed by a casing (6) compartmentalized by a separating element (7), which may be a filter, forming the separation between an upper section (8) where the emanating material (2) is located and a lower air chamber (9) where there is coupled a nozzle (10) in which there are defined exit openings (3) through which the odor exits to be tested. It also comprises a support (4) and adherence means (5), for example a magnet, connecting the body (1) to the support (4) such that the exit openings (3) are covered by the support (4) preventing the exit of the odor until a new testing which is done when the body (1) is separated from the support (4).

## Description

### OBJECT OF THE INVENTION

The present invention relates to a device for presenting smells which maintain the aroma of the integral odorous composition for a very long time, which does not allow spillage and uses a sealing system without the specific conventional handling for covering it.

The object of the invention is for the device for testing odors to comprise a small and easy-to-handle body emanating a characteristic odor having openings for the exit of the odor and combined with a support on which the body rests to provide the closure of the openings, the odor being released once the body is lifted and separated from the support.

### BACKGROUND OF THE INVENTION

There are currently few systems for testing odors, the most common of which being small perfume sample bottles with a pressure or screw-on cap, and those having a safety plug installed under pressure on the mouth of the bottle which opens directly to be able to smell it. These plugs are usually difficult to extract and hands frequently become wet when opening the bottle and are impregnated with the odor.

Blotting paper is also known and has an elongated configuration with a width of 4 to 10 mm and a length of 10 to 200 mm, generally ending in a point which is wetted in the liquid contained in a bottle, maintaining the complete odor for a limited time.

To elongate the duration of the odor, the drying papers are placed inside special waxed and odorless envelopes, however they have the drawback that the more volatile aromas quickly disappear in very little time even inside the envelope, and the complete perception of the odorous composition is lost. Odor drying papers are useful for perfumers for the evaluation of the evaporation steps of a perfume but not for perceiving the constancy of an odor.

On the other hand it is necessary to point out the existence of an apparatus for smelling substances in an individual manner described in utility model ES 1,029,514 belonging to the same proprietor of the present invention, which is intended for carrying out tests for smelling perfumes, aromas, air fresheners, consisting of a translucent and leak-tight container internally having a bottle containing the odorous substance and at a certain distance above the bottle contains a rotating shaft transversely assembled and joined to a laminar body which leads the odor to the exterior when it rotates through openings defined on its upper face.

### DESCRIPTION OF THE INVENTION

The device for smelling odors constituting the object of this invention comprises a preferably small odor testing element consisting of a body emanating a characteristic odor in combination with a support to which the body is provisionally adhered and more specifically by means of a face provided with openings resting on the support, preventing the odor from coming out. Once the body is lifted from the support, the openings are freed and the odor can be perceived through such openings.

The body can be made of any material which does not chemically interact with the odorous substances or if the interaction exists it should not be relevant, using for example wood, metals, noble metals, synthetic materials or the like.

The body is hollow, completely closed and allows the air to enter and exit through the openings so as to allow the emanation of the odor contained therein.

The body internally has a porous and absorbent material containing the odorous substance which is separated from the area in which the openings are located by a space where a certain volume of the complete odorous composition in a gaseous state is confined. A separation is therefore arranged in the body by means of a separating element, which may consist of a filter, for example, between an upper chamber in which the porous material is housed and a lower air chamber transferring the odor through the openings towards the exterior.

The liquid amount of the porous material with respect to the total volume of the body allows creating saturation inside the body with said substance in the gaseous state, thus establishing dynamic equilibrium between the odorous substance in the liquid state and gaseous state, complying with the premise that the odor exiting through the openings when the body is brought closer to the nose is the complete odorous composition.

The body internally has a magnet or magnetized surface close to the openings to facilitate its adherence or separation with respect to the support base, providing the tight seal of the system preventing the odor for exiting to the exterior.

The body can be easily removed by separating it from the support base using the fingers with minimal effort, bringing it close to the nose, and the odor exits through the openings at the same time it is separated from the support due to the fact that it contains a saturation of the odorous substance in the gaseous state close to the openings.

The perception of the odor becomes greater in a few seconds due to the increase produced by the effect of the temperature of the fingers in contact with the metallic body which quickly transmits the vapor pressure of the odorous substance and therefore its exit through the openings.

The support provided essentially consists of a planar base of a ferruginous material, the possibility of said support constituting the base of a box provided with a cover being provided, providing better conditions for closure, as well as for the transport and presentation of the device.

It is also contemplated that instead of having a planar base, the body can have a recess in its base which facilitates its adaptation to the support in cooperation with a projection having the same geometry defined in such support, improving the fixing conditions.

This device for smelling odors has several different applications, and it can especially be used as a promotional element in advertising campaigns for certain products for effectively smelling the product since it allows maintaining the complete odor for a long period of time invariably.

Its application for presenting primary or final odors associated to wines, cheeses, oils, waters and the like must be pointed out, and it could also constitute a practical and effective system for presenting perfumes and aromas.

Other applications could be related to its use for a reminder or for the transmission of information for the blind, and in efficacy and creativity studies in the interaction of the senses.

It could also be used as a corporate odorous identity seal, in jewelry for the use of noble metals with an odor, in odorous sculptures, in odor guessing games, in refrigerator magnets, in the olfactory testing complementary to eating foods.

### DESCRIPTION OF THE DRAWINGS

To complement the description being made and for the purpose of aiding in better understanding the features of the invention according to a preferred practical embodiment thereof, a set of drawings is attached as an integral part of said description in which the following has been shown with an illustrative and non-limiting character:

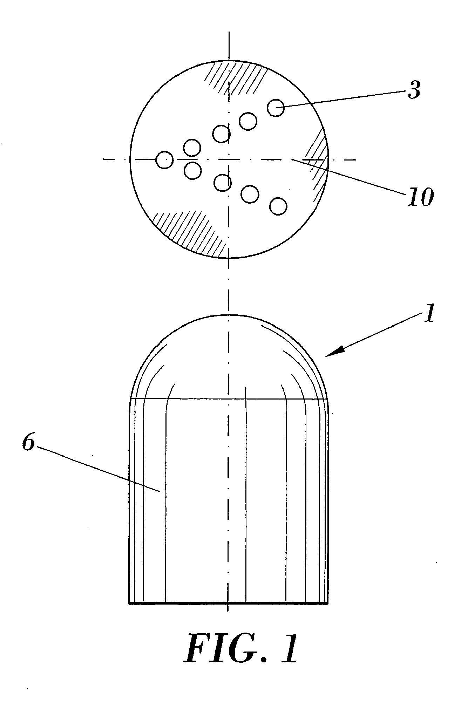

Figure 1 shows an elevated and lower view of the face of the device for smelling odors having the through openings.

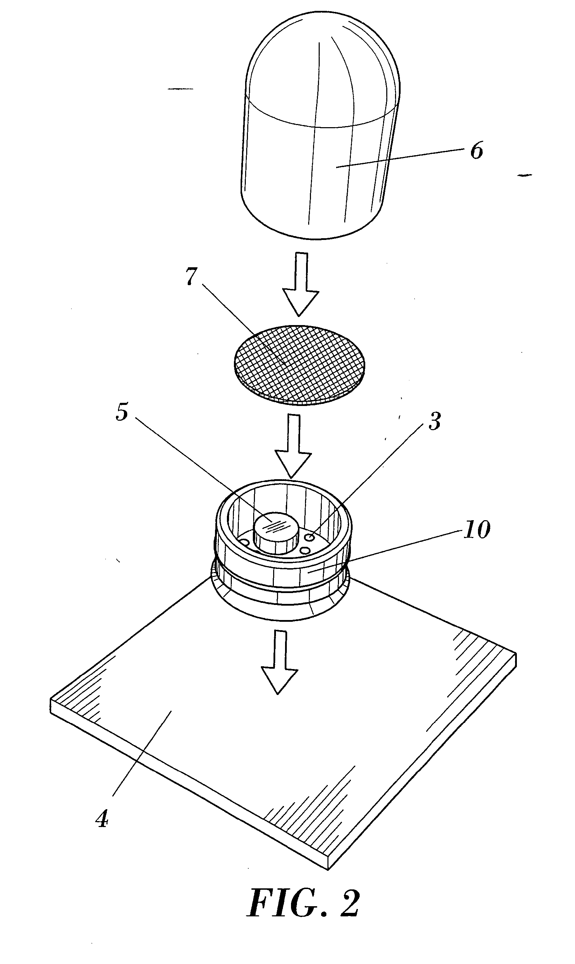

Figure 2 shows an exploded view of the device object of this invention in which the elements forming it are observed.

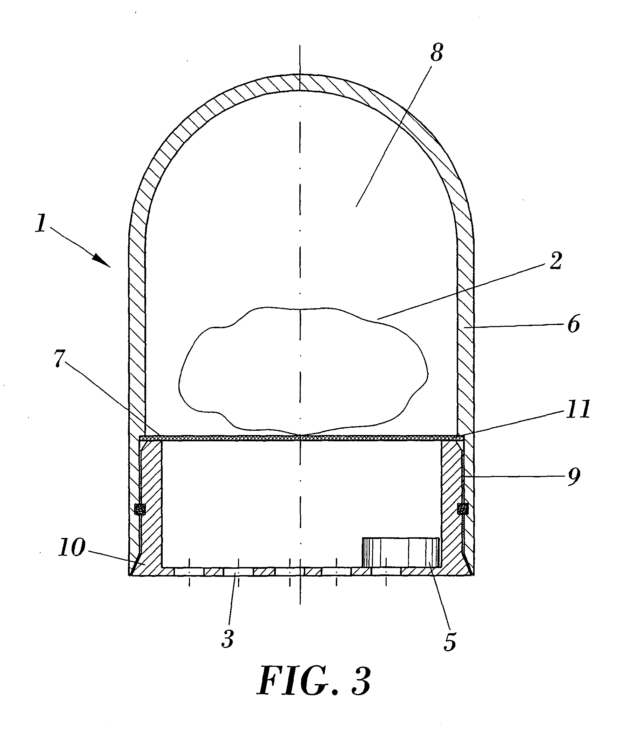

Figure 3 shows a sectional view of the body of the device for smelling odors.
Figure 4 shows a perspective view of the odor testing device with the support base forming part of a box provided with a cover, the body being seen inside the box with the cover partially open.
Figure 5 shows a lower perspective view of an alternative embodiment of the device in which a space is defined between the nozzle and the perimetric edge of the body defining a recess in its base, and the support with a projection having a geometry complementary to the recess is seen.
Figure 6 shows a perspective view of the device located at the end of an elongated body simulating the stem of a flower.

### PREFERRED EMBODIMENT OF THE INVENTION

The device for smelling odors constituting the object of this invention comprises a body (1) internally incorporating an emanating material (2) emanating a characteristic odor, and has on one of its faces exit openings (3) through which the odor exits to be tested, and it also comprises a support (4) and adherence means (5) for adhering the body (1) to the support (4) which determine that the exit openings (3) are covered by the support (4) preventing the exit of the odor' until a new testing which is done when the body (1) is lifted and separated from the support (4).

The body (1) is formed by a casing (6) compartmentalized by a separating element (7) provided with through apertures for the odor which forms the separation between an upper section (8) where the emanating material (2) is located and a lower air chamber (9) where there is coupled a nozzle (10) in which the exit openings (3) are defined.

The separating element (7) may consist of a filter or any other part provided with through apertures allowing the passage of the odor towards the air chamber (9).

Defined inside the body (1) there is a step (11) where the separating element (7) rests, pressed against by the nozzle (10) which is inserted inside the body (1).

It has been provided that the adherence means (5) consist of a magnet located in the nozzle (10) inside the face incorporating the exit openings (3) and that the support (4) comprises a ferruginous material.

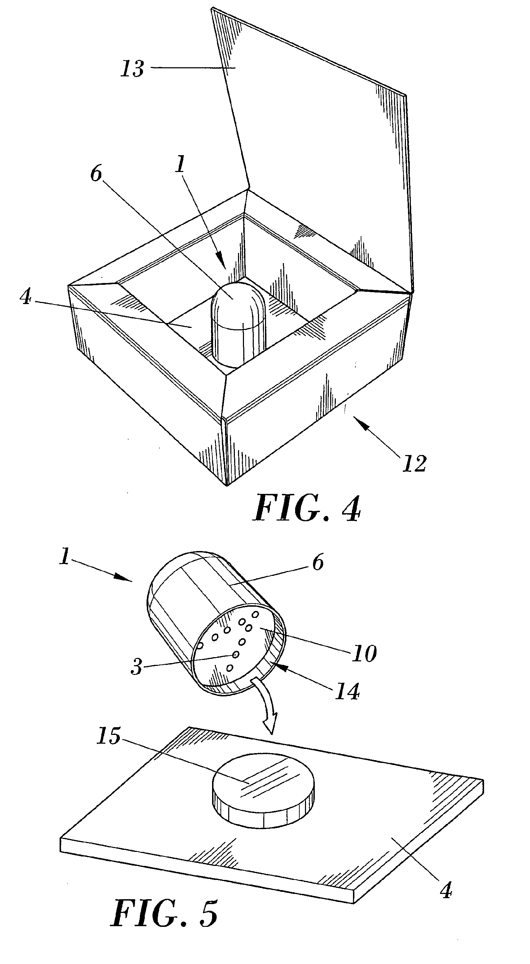

On the other hand the support (4), as shown in Figure 4, may form the lower base of a box (12) of the type having a collapsible upper cover (13) keeping the box (12) closed.

The closure of the upper cover (13) can be arranged by corresponding fixing means that may consist of a magnet inside the cover (13) or inside the body of the box (12) and the corresponding ferruginous body incorporated on the other surface.

Figure 5 shows an alternative embodiment of the body (1) in which it can be seen that the base of the nozzle (10) where the openings (3) are located is spaced and recessed inside the body (6) defining a broad recess (14) at its base, and corresponding with such recess the support (4) has a projection (15) with a coinciding geometry facilitating the fixing of the body (1).

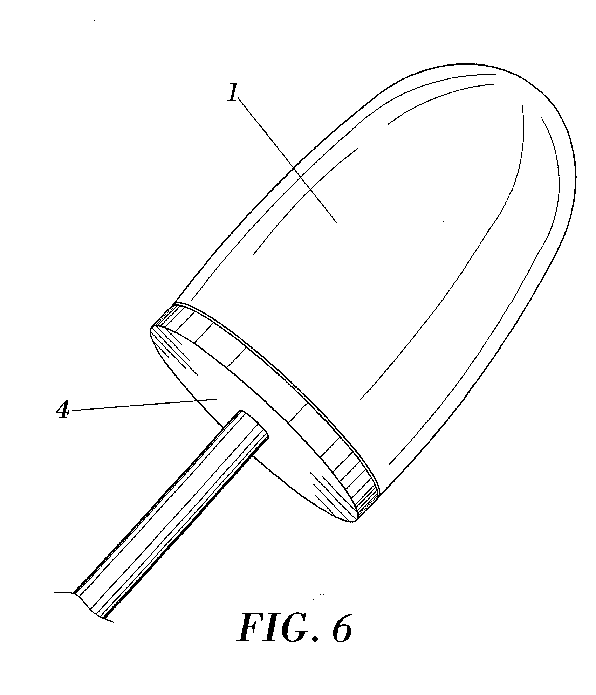

The support (4) on which the body (1) is located may at least have a diameter or width, depending on the shape of the body (1), that is equal to the shape corresponding to the base of the body (1), the possibility being contemplated that a type of stem extends from the support (4), as can be seen in Figure 6, and that the body (1) has any possible shape, such as the shape of a flower, for example a rose, or any decorative or gift object, or maintaining the same design represented in the previous figures, containing an aroma of a flower, for example.

## Claims

1. A device for smelling odors **characterized in that** it comprises a body (1) internally incorporating an emanating material (2) emanating a characteristic odor, and has on one of its faces exit openings (3) through which the odor exits to be tested, and it also comprises a support (4) and adherence means (5) for adhering the body (1) to the support (4) determining that the exit openings (3) are covered by the support (4) preventing the exit of the odor until a new testing which is done when the body (1) is lifted and separated from the support (4).

2. A device for smelling odors according to claim 1 **characterized in that** the body (1) is formed by a casing (6) compartmentalized by a separating element (7) provided with through apertures for the odor forming the separation between an upper section (8) where the emanating material (2) is located and a lower air chamber (9) where there is coupled a nozzle (10) in which the exit openings (3) are defined.

3. A device for smelling odors according to the previous claims, **characterized in that** defined inside the body (1) there is a step (11) where the separating element (7) rests, pressed against by the nozzle (10).

4. A device for smelling odors according to the previous claims, **characterized in that** the separating element (7) consists of a filter.

5. A device for smelling odors according to claims 1 and 3, **characterized in that** the adherence means (5) consist of a magnet located in the nozzle (10) inside the face incorporating the exit openings (3) and the support (4) comprises a ferruginous material.

6. A device for smelling odors according to claims 1 and 5, **characterized in that** the support (4) forms the lower base of a box (12) having a collapsible upper cover (13) keeping the box (12) closed.

7. A device for smelling odors according to claims 1, 2, 5 and 6, **characterized in that** the base of the nozzle (10) where the openings (3) are located is spaced and recessed inside the body (6) defining a broad recess (14) at its base, and corresponding with such recess the support (4) has a projection (15) with a coinciding geometry facilitating the fixing of the body (1).
